# FASCICULE DE BREVET EUROPEEN

(11) **EP 0 722 460 B1**
(45) Date de publication et mention de la délivrance du brevet: **08.09.1999**
(21) Numéro de dépôt: 94929585.1
(22) Date de dépôt: 06.10.1994
(51) Int. Cl.: C07K 7/08, A61K 38/58

(54) **DERIVES DE PEPTIDES THERAPEUTIQUEMENT ACTIFS DANS LA CASCADE DE COAGULATION SANGUINE, LEUR PROCEDE DE PREPARATION ET LES COMPOSITIONS PHARMACEUTIQUES QUI LES CONTIENNENT**
DERIVATE VON THERAPEUTISCH IN DER BLUTGERINNUNGSKASKADE WIRKSAMEN PEPTIDEN, VERFAHREN ZUR HERSTELLUNG UND SIE ENTHALTENDE PHARMAZEUTISCHE ZUBEREITUNGEN
DERIVATIVES OF PEPTIDES THERAPEUTICALLY ACTIVE IN THE CASCADE SEQUENCE FOR BLOOD COAGULATION, PROCESS FOR THEIR PREPARATION AND PHARMACEUTICAL COMPOSITIONS CONTAINING SAME

(30) Priorité: 07.10.1993 FR 9311942
(43) Date de publication de la demande: 24.07.1996
(73) Titulaire: ADIR ET COMPAGNIE, 92415 Courbevoie Cédex (FR)
(72) Inventeur: FAUCHERE, Jean-Luc, F-92210 Saint-Cloud (FR); THURIEAU, Christophe, F-75116 Paris (FR); VERBEUREN, Tony, F-78540 Vernouillet (FR); RUPIN, Alain, F-37510 Savonnières (FR); SIMONET, Serge, F-78700 Conflans-Sainte-Honorine (FR)
(86) Numéro de dépôt international: FR9401166
(87) Numéro de publication internationale: WO9509869

(56) Documents cités:
- EP-A- 0 552 999
- BIOCHEMISTRY., vol.29, no.30, 31 Juillet 1990, EASTON, PA US pages 7095 - 7101 J. M. MARAGANORE ET AL. 'Design andcharacterization of hirulogs; a novel class of bivalent peptide inhibitors of thrombine'
- FEBS LETTERS., vol.294, no.3, 9 Décembre 1991, AMSTERDAM NL pages 163 - 166 P. BOURDON ET AL. 'Structure-function relationships of hirulog peptide interactions with thrombin'
- THROMBOSIS & HAEMOSTASIS, vol.63, no.2, 1990, STUTTGART, DE pages 208 - 214 J. L. KRSTENANSKY ET AL. 'Development of MDL 28, 050, a small stable antithrombin agent based on a functional domain of the leech protein, hirudin'
- HELVETICA CHIMICA ACTA, vol.77, no.3, 11 Mai 1994, BASEL CH pages 679 - 684 C.THURIEAU ET AL. 'Synthesis of a new bivalent hirudin analogue (hirufos), which includes a stable 4'-phosphono-L-phenylalanine mimic of (L-tyrosine O4-sulfate)'

## Description

La présente invention concerne de nouveaux dérivés de peptides thérapeutiquement actifs dans la cascade de coagulation sanguine, leur procédé de préparation et les compositions pharmaceutiques qui les contiennent.

Il est désormais largement connu que lorsque l'équilibre entre facteurs procoagulants et anticoagulants dans le sang est perturbé, il peut en résulter la formation d'un thrombus ou caillot de sang. Le développement d'une thrombose est favorisé essentiellement par trois facteurs pathogéniques principaux qui sont la stase ou diminution du flux sanguin, les états d'hypercoagulabilité et les lésions de l'endothélium de la paroi vasculaire. Contre ces pathogénies, il convient donc d'établir un traitement dont l'une des bases principales est le médicament anticoagulant.

Les anticoagulants sont en effet utilisables dans le traitement des thromboses veineuses aigües, de l'embolie pulmonaire, de l'embolie artérielle des extrêmités, des thromboses artérielles comme l'infarctus du myocarde, de l'athérosclérose, de toutes les autres manifestations thromboemboliques ainsi que pour maintenir l'homéostasie sanguine, en particulier dans la circulation extracorporelle.

Parmi les agents anticoagulants connus, l'hirudine, qui est un polypeptide comportant 65 acides aminés, est un inhibiteur spécifique de la thrombine isolé des glandes salivaires de la sangsue médicinale (Biochemistry 25, 4622-28, 1986).

Des variantes de l'hirudine utilisables en tant qu'inhibiteur de thrombine ont déjà été décrits. C'est le cas, par exemple, des composés décrits dans les brevets EP 209061 ou EP 332523. D'autre part, des analogues synthétiques de fragments d'hirudine à propriétés anticoagulantes ont également été décrits ; c'est le cas, par exemple, des composés revendiqués dans les brevets EP 276014, EP 291981, EP 291982 et EP 333356. Par rapport au modèle naturel, ces fragments plus courts (10 à 20 acides aminés) offrent l'avantage d'être plus "maniables" : en particulier leur synthèse est plus simple.

Plus récemment, la demande de brevet européen EP 0372503 a revendiqué des peptides analogues de l'hirudine dans lesquels un acide aminé naturel était remplacé par un acide aminé synthétique.

La demande EP 0443598 revendique des peptides analogues de l'hirudine dans lesquelles un acide aminé naturel est remplacé par un dérivé sulfoné ou phosphoné.

Les demandes PCT 91/01328 et EP 443429 revendiquent des analogues de l'hirudine dans lesquels les modifications portent à la fois sur l'introduction d'amino-acides non naturels mais également sur l'introduction de sulfono-oxo ou phosphono-oxo-amino-acides.

La présente invention concerne plus particulièrement les composés de formule (I) : H-D-Phe-A1-Arg-Pro-(Gly)4-Asn-Gly-Asp-Phe-Glu-Abo-Ile-Pro-Glu-Glu-A2-Leu-D-Glu-OH (I) dans laquelle :
- A₁: représente un résidu proline (Pro), octahydroindole-2-carbonyle (Oic), 2-azabicyclo[2.2.1]heptane-3-carbonyle (Abh) ou 2-azabicyclo [2.2.2]octane-3-carbonyle (Abo),
- A₂: représente un résidu phénylalanine substitué en para ou en méta par un groupement PO₃H₂(Phe(pPO₃H₂), Phe(mPO₃H₂)),
leurs sels d'addition à un acide ou à une base pharmaceutiquement acceptable, chaque amino-acide de la séquence peptidique étant optiquement pur et le carbone a de chaque amino-acide ayant la configuration D ou L.

Parmi les acides pharmaceutiquement acceptables on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables on peut citer à titre non limitatif l'hydroxyde de sodium, l'hydroxyde de potassium, la triéthylamine, la tertbutylamine, etc...

L'invention s'étend aussi au procédé de préparation des dérivés de formule (I) qui peuvent être obtenus par différentes méthodes telles que la synthèse séquentielle sur phase solide, la synthèse de fragments et leur couplage en solution, la synthèse enzymatique, la synthèse génétique par clonage et expression de gènes dans des bactéries transformées ou par des combinaisons diverses de ces techniques.

Les méthodes générales de la synthèse des peptides sur phase solide ont été décrites par B.W. ERICKSON et R.B. MERRIFIELD ("The Proteins", Solid-Phase Peptide Synthesis, 3^{ème} édition, 257-527, 1976).

La synthèse sur phase solide peut se réaliser sur un automate qui effectue de façon répétitive et programmable des cycles de déprotection, couplage et lavages nécessaires à l'introduction séquentielle des acides aminés dans la chaîne peptidique. L'acide aminé, de préférence C-terminal est fixé sur une résine conventionnellement utilisée pour la préparation de polypeptides, de préférence un polystyrène réticulé à l'aide de 0,5 à 3,0 % de divinylbenzène et muni de restes activés tels que le chlorométhylène ou l'hydroxyméthylène qui permettent la fixation covalente du premier acide aminé sur la résine. Le choix approprié de la résine permet la fixation d'une fonction C-terminale acide carboxylique, amide ou alcool.

Les acides aminés sont alors introduits un à un dans l'ordre déterminé par l'opérateur. Chaque cycle de synthèse correspondant à l'introduction d'un acide aminé, comporte une déprotection, de préférence N-terminale de la chaîne peptidique, des lavages successifs destinés à éliminer les réactifs ou à gonfler la résine, un couplage avec activation de l'acide aminé et de nouveaux lavages. Chacune de ces opérations est suivie d'une filtration réalisée grâce à la présence d'un verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBT) ou le benzotriazol-1-yl-oxytris (diméthylamino) phosphonium hexafluorophosphate (BOP) ou encore le diphenylphosphorylazide (DPPA).

Des activations par formation d'anhydrides mixtes sont également possibles.

Chaque acide aminé est introduit dans le réacteur en quadruple excès environ, par rapport au degré de substitution de la résine et en quantité environ équivalente par rapport aux agents de couplage. La réaction de couplage peut être vérifiée à chaque étape de la synthèse par le test de réaction à la ninhydrine décrit par E. KAISER et Coll. (Analyt. Biochem., 34, 595, 1970).

Après assemblage de la chaîne peptidique sur la résine, un traitement par un acide fort tel que l'acide trifluoroacétique, ou l'acide fluorhydrique en présence d'anisole, d'éthanedithiol ou de 2-méthylindole sert à séparer le peptide de la résine ainsi qu'à libérer éventuellement le peptide de ses groupes protecteurs. Le composé est alors purifié par des techniques classiques de purification, notamment chromatographiques.

Les peptides de la présente invention peuvent être également obtenus par couplage en solution de fragments peptidiques sélectivement protégés, qui peuvent être préparés soit sur phase solide, soit en solution. L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide. Les méthodes d'activation lors des couplages sont également analogues à celles employées dans la synthèse sur phase solide.

Les composés de formule (I) possèdent des propriétés pharmacologiques très intéressantes. Ils sont doués de propriétés anticoagulantes et antithrombotiques et peuvent ainsi être utilisés pour prévenir les complications post-thromboemboliques par dissolution des caillots ou en tant qu'agents préventifs de l'extension du processus thrombotique en les utilisant comme anticoagulants d'action directe et rapide. Les résultats obtenus lors des essais pharmacologiques montrent que les composés de l'invention possèdent une activité antithrombotique largement supérieure à celle d'un composé de référence, l'hirulog-1 (J.M. MARAGANORE & coll., Biochem., 29, 7095-7101, 1990). Cette meilleure activité est due à une inhibition plus importante du site catalytique de la thombine.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols, ampoules buvables, injectables, etc...

La posologie varie selon l'âge et le poids du patient, la nature et la sévérité de l'affection ainsi que la voie d'administration.

Celle-ci peut être orale, nasale, rectable ou parentérale. D'une manière générale, elle s'échelonne entre 0,05 et 30 mg pour un traitement en une ou plusieurs prises par 24 heures.

Les exemples suivants illustrent l'invention et ne la limitent en aucune façon.

Dans les exemples ci-dessous, les acides aminés en général sont de configuration L. Les acides aminés de configuration D sont identifiés par la lettre D.

L'acide aminé nommé Abo est de configuration 3S.

L'acide aminé nommé Oic est de configuration 2S,3aS,7aS.

Phe (pPO₃H₂) représente le résidu :

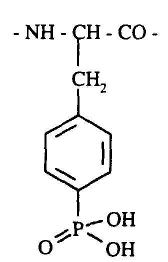

Phe (mPO₃H₂) représente le résidu :

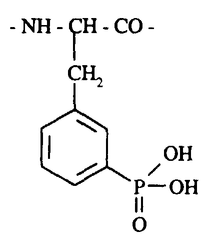

Le composé décrit dans la préparation A est un intermédiaire utile dans la synthèse des composés de formule (I).

### PREPARATION A : N-Fmoc-4-(diméthoxyphosphoryl)-L-phénylalanine (ou Fmoc-Phe(pPO₃(Me)₂)

### STADE A : N-Boc-4[((trifluorométhyl)sulfonyl)oxy]-L-phénylalanine benzylester

A une solution contenant 6,8 mmoles de N-Boc-L-tyrosine benzylester et 7,4 mmoles de phényl bis((triluorométhyl)sulfonyl)amine dans 10 ml de dichlorométhane refroidie dans un bain de glace, sont ajoutées goutte à goutte 7,4 mmoles de triéthylamine. Le mélange est agité 1 heure à 0°C et 2 heures à température ambiante. Après addition de 60 ml de diéthyléther, la phase organique est lavée à l'eau, à la soude IN, à l'eau, puis par une solution saturée de chlorure de sodium, séchée et évaporée. Le produit attendu est alors obtenu sous forme d'huile, après purification par chromatographie sur colonne de silice en utilisant comme éluant un mélange pentane/acétate d'éthyle (80/20).

### STADE B : N-Boc-4-(diméthoxyphosphoryl)-L-phénylalanine benzylester

Une suspension contenant 1,1 mmoles du composé obtenu au stade précédent, 1,4 mmoles de méthylmorpholine, 1,32 mmoles de (EtO)₂POH et 0,032 mmole de Pd(O)(pPH₃)₄ dans 3 ml d'acétonitrile anhydre, est agitée à 70°C, sous atmosphère d'azote, pendant 15 heures. Après refroidissement, on ajoute 40 ml d'acétate d'éthyle. La phase organique est lavée par de l'acide citrique à 5 %, par une solution d'hydrogénocarbonate de sodium à 10 % puis séchée et évaporée. Le produit attendu est alors obtenu, sous forme d'huile, après purification par chromatographie sur colonne de silice en utilisant comme éluant un mélange acétate d'éthyle/pentane (3/7).

### STADE C : N-Boc-4-(diméthoxyphosphoryl)-L-phénylalanine

Une solution contenant 14,4 mmoles du composé obtenu au stade précédent dans 60 ml de méthanol et 40 ml d'eau est réduite sous pression d'hydrogène en utilisant du palladium/charbon à 10 % comme catalyseur. Après filtration et évaporation du solvant, on obtient le produit attendu sous forme d'huile.

### STADE D : Fmoc-Phe(pPO₃(Me)₂)

Le composé obtenu au stade précédent est déprotégé selon la technique décrite par B. GUTTE et coll. (J. Biol. Chem., 246, 1922-1941, 1971) et le groupement Fmoc est introduit selon la technique décrite par Carpino et coll. (J. Org. Chem., 37, 3404-3409, 1972).

### EXEMPLE 1 :

H-D-Phe-Pro-Arg-Pro-(Gly)4-Asn-Gly-Asp-Phe-Glu-Abo-Ile-Pro-
(Glu)2-Phe(pPO3H2)-Leu-D-Glu-OH

Le composé de l'exemple 1 est synthétisé à partir de 2 g d'une résine substituée par 0,33 mmole/g de Fmoc-glu(OtBu)-OH et suivant le protocole répétitif suivant :

| N° opération | Fonction | Solvant/Réactif | Répétition/temps |
|---|---|---|---|
| 1 | lavage | DMF | 2 x 2 min |
| 2 | déprotection | 20 % pipéridine/DMF | 1 x 5 min |
| 3 | déprotection | 20 % pipéridine/DMF | 1 x 15 min |
| 4 | lavage | DMF | 3 x 2 min |
| 5 | lavage | dichlorométhane | 3 x 2 min |
| 6 | couplage | acide aminé protégé activé | 1 x 90 min |
| 7 | lavage | DMF | 3 x 2 min |
| 8 | lavage | isopropylique alcool | 3 x 2 min |
| 9 | lavage | dichlorométhane | 3 x 2 min |

Chacune de ces opérations effectuée dans 30 ml de solvant, sous agitation et à température ambiante, est suivie d'une filtration à travers un verre fritté incorporé à la cellule de verre (réacteur) dans laquelle la synthèse progresse. Le filtre retient la résine sur laquelle est fixée la chaîne peptidique croissante.

Les acides aminés protégés choisis ont été introduits dans l'ordre suivant :

Fmoc-Leu-OH, Fmoc-Phe(pPO3Me2)-OH, Fmoc-Glu(OtBu)-OH, Fmoc-Glu(OtBu)-OH,
Fmoc-Pro-OH, Fmoc-Ile-OH, Fmoc-Abo-OH, Fmoc-Glu(OtBu)-OH, Fmoc-Phe-OH,
Fmoc-Asp(OtBu)-OH, Fmoc-Gly-OH, Fmoc-Asn-OH, Fmoc-Gly-OH, Fmoc-Gly-OH,
Fmoc-Gly-OH, Fmoc-Gly-OH, Fmoc-Pro-OH, Fmoc-Arg(Pmc)-OH, Fmoc-Pro-OH,
Fmoc-phe-OH.

L'activation en vue du couplage (opération 6) est obtenue à chaque cycle par dissolution de 4 équivalents (2.64 mmoles) de l'acide aminé protégé avec 360 mg d'HOBt dans 30 ml de DMF, puis après 30 minutes à température ambiante, par addition de 618 mg de DCC. Cette solution est alors immédiatement introduite dans la cellule de réaction avec 10 ml de dichlorométhane.

Le produit attendu est alors obtenu après traitement classique de la résine par de l'acide trifluoroacétique (TFA) à 95 % en présence d'anisole et d'éthanedithiol suivi d'une déprotection sélective des méthyles du groupement phosphate par une solution contenant 90 % de TFA, 5 % de (CH₃)₃SiBr et 5 % de thioanisole. Le peptide est alors purifié par CLHP préparative et lyophilisé.

L'analyse du produit obtenu est réalisée après décomposition de celui-ci en acides aminés par hydrolyse dans de l'acide chlorhydrique 6N pendant 18 heures à 110°C et dosage quantitatif des acides aminés obtenus par CLHP.

| | | | | | | | | |
|---|---|---|---|---|---|---|---|---|
| | Arg | Gly | Asp+Asn | Phe | Glu | Abo+Ile | Pro | Leu |
| calculé | 1 | 5 | 2 | 2 | 4 | 2 | 3 | 1 |
| trouvé | 1,04 | 4,9 | 2,01 | 1,89 | 4,27 | 1,82 | 3,02 | 1,03 |

Spectre de masse (FAB) : MH⁺, m_{/z} = 2381

Les exemples suivants ont été préparés en utilisant le procédé décrit dans l'exemple 1.

### EXEMPLE 2 :

H-D-Phe-Oic-Arg-Pro-(Gly)4-Asn-Gly-Asp-Phe-Glu-Abo-Ile-Pro-
(Glu)2-Phe(pPO3H2)-Leu-D-Glu-OH

| | | | | | | | | | |
|---|---|---|---|---|---|---|---|---|---|
| | Arg | Asp+Asn | Glu | Gly | Pro | Phe | Abo+Ile | Leu | Oic |
| calculé | 1 | 2 | 4 | 5 | 2 | 2 | 2 | 1 | 1 |
| trouvé | 0,87 | 2,08 | 4,31 | 5,07 | 2,13 | 1,82 | 1,89 | 1,08 | 1,03 |

Spectre de masse (FAB) : MH⁺, m_{/z} = 2435

### EXEMPLE 3 :

H-D-Phe-Abh-Arg-Pro-(Gly)4-Asn-Gly-Asp-Phe-Glu-Abo-Ile-Pro-
(Glu)2-Phe(pPO3H2)-Leu-D-Glu-OH

### EXEMPLE 4 :

H-D-Phe-Abo-Arg-Pro-(Gly)4-Asn-Gly-Asp-Phe-Glu-Abo-Ile-Pro-
(Glu)2-Phe(pPO3H2)-Leu-D-Glu-OH

### EXEMPLE 5 :

H-D-Phe-Pro-Arg-Pro-(Gly)4-Asn-Gly-Asp-Phe-Glu-Abo-Ile-Pro-
(Glu)2-Phe(mPO3H2)-Leu-D-Glu-OH

### ETUDE PHARMACOLOGIQUE DES COMPOSES DE L'INVENTION

### EXEMPLE 6 : Activité antithrombotique

Le modèle de thrombose expérimentale utilisé est celui d'une thrombose veineuse induite chez le rat OFA mâle par ligature de la veine cave inférieure, (Millet et coll., Thrombosis Res., 45 : 123-133, 1987). Les animaux à tester sont anesthésiés par administration de pentobarbital sodique (50 mg/kg i.p.). 15 minutes avant la ligature, les animaux sont traités avec les produits à tester par voie i.v. Le caillot est retiré de la veine cave 25 minutes après la ligature, il est pesé après dessiccation (24 heures à 60°C). Les composés de l'invention ont été testés aux doses de 0,05, jusqu'à 1 mg/kg et comparés à l'hirulog-1 (0,1 et 1 mg/kg). Les résultats de ce test montrent que les composés de l'invention permettent une diminution signification du poids du caillot plus important que celle observée avec le composé de référence, l'hirulog-1 : le composé de l'exemple 1 est 20 fois plus actif et le composé de l'exemple 2 est 10 fois plus actif que l'hirulog-1 sur ce modèle.

| Diminution du poids de caillot en % : | | | | |
|---|---|---|---|---|
| **Dose (mg/kg)** | **0,05** | **0,1** | **0,5** | **1** |
| hirulog-1 | | - 0% | | - 46 % |
| Exemple 1 | - 45 % | - 64 % | - 71 % | - 83 % |
| Exemple 2 | | - 45 % | - 62 % | |

### EXEMPLE 7: Activité anticoagulante, mesure du temps de thrombine et de temps de céphaline activée

En présence d'une quantité standard de thrombine ou de céphaline activée, un plasma normal coagule en un temps défini et constant appelé respectivement temps de thrombine (TT) et temps de céphaline activée (TCA). L'allongement de ces temps de coagulation par des substances pharmacologiques traduit un effet anticoagulant.
L'effet in vitro des produits de l'invention a été mesuré sur le plasma humain. Le sang a été prélevé par ponction veineuse dans un tube contenant du citrate. Après décantation (1200 g, 10 min) le plasma pauvre en plaquettes est décanté pour réaliser ensuite les tests de coagulation. Les composés de l'invention allongent de façon significative le TT et le TCA. Le composé de l'exemple 1 double le TT à la concentration de 0,05 µM et le TCA à la concentration de 0,14 µM. Cette activité est plus importante que celle de l'hirulog-1, respectivement 0,07 µM et 0,2 µM.
L'effet des produits a été testé ex vivo chez le rat OFA anesthésié avec du pentobarbital sodique (60 mg/kg i.p.). L'artère carotide et la veine jugulaire sont cathétérisées. Après installation des cathéters, un prélèvement de 1,5 cm³ de sang artériel est effectué sur citrate (0,109 M). 30 minutes après, le produit à tester est administré sous un volume de 1 ml en i.v. Des prélèvements artériels (1,5 cm³) sont alors effectués à 15 min, 3 min, 5 min, 15 min, 30 min et 60 min. A chaque prélèvement, 1,5 cm³ de sérum physiologique citraté est réinjecté à l'animal via la carotide. Les composés de l'invention augmentent de façon significative le TT et le TCA ex vivo chez le rat. Ils augmentent le TT (facteur multiplicatif) et le TCA (facteur multiplicatif) de manière plus importante que l'hirulog-1 et la durée d'action est plus importante.

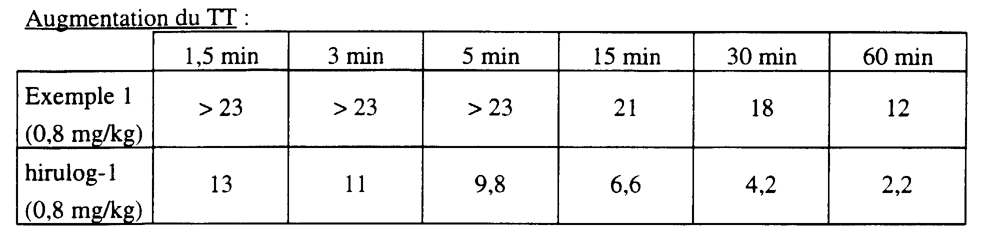

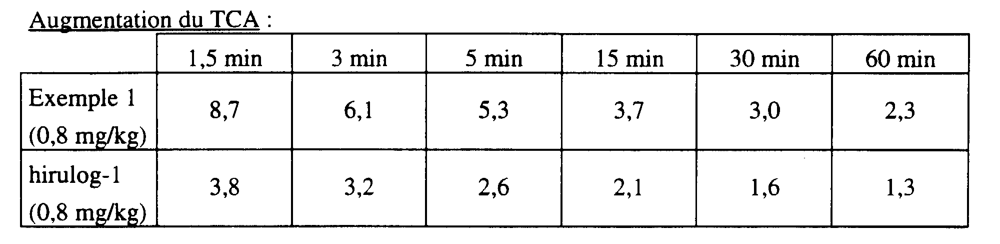

### EXEMPLE 8 : Inhibition de la thrombine et des sérines protéases de la coagulation

Pour évaluer in vitro l'activité inhibitrice des produits de l'invention sur la thrombine humaine (Sigma, activité spécifique 3230 UNIH/mg), le fibrinogène humain (purifié ou plasmatique) (Fg) ou le substrat chromogène H-D-Phe-Pip-Arg-pNa (0,66 nM, S 2238, Kabi) ont été ajoutés à une quantité donnée de thrombine (0,7 ou 2 nM) préalablement incubée avec ou sans l'inhibiteur à tester (20°C, 10 min).
Pour évaluer in vitro la sélectivité de ces produits vis-à-vis de différentes sérines protéases de la coagulation, le même protocole a été appliqué à la plasmine humaine purifié (2 nM, Stago), au facteur X activé humain purifié (2 nM, Stago), à la kallikréine (2 nM, Sigma), à l'urokinase (2 nM, Sigma), à l'activateur tissulaire du plasminogène (t-PA ; 2 nM, Stago) et à la protéine C activée humaine purifiée (2 nM, Stago) en utilisant pour substrats différents peptides parnitroanilidés.
Inhibiteurs, enzymes et substrats sont dilués dans le même tampon (tampon phosphate 0,01 mM, pH 7,4, contenant 0,12 M de chlorure de sodium et 0,05 % de sérum albumine bovine) puis distribués dans une microplaque en polystyrène sous un volume de 50 µl.
La fibrine formée par la thrombine ou le paranitroanilide libéré par l'action de la sérine protéase est mesurée spectrophotométriquement à 405 nm après 15 à 30 minutes de réaction à 20°C.
Les résultats présentés ci-dessous montrent l'inhibition de la thrombine par les substances de l'invention en comparaison avec l'hirulog-1 vis-à-vis du fibrinogène et du substrat chromogène. Les résultats sont exprimés en valeur IC₅₀ : la concentration en nM qui inhibe de 50 % l'activité enzymatique. Les composés de l'invention ont une activité supérieure à celle de l'hirulog-1. Ceci est particulièrement le cas en présence du substrat chromogène démontrant une activité inhibitrice supérieure des produits de l'invention sur le site catalytique de la thrombine.

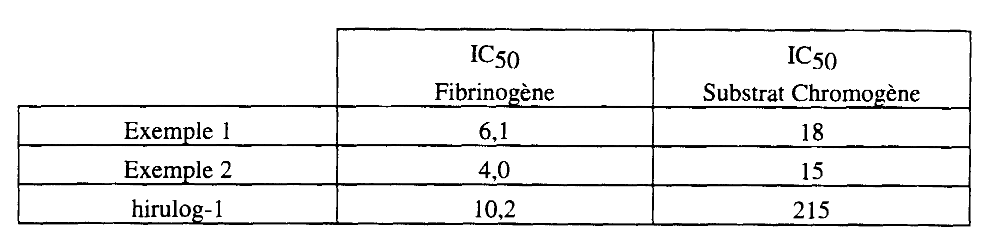

Comme l'hirulog-1, les produits de l'invention sont extrêmement sélectifs de la thrombine puisque les IC₅₀ vis-à-vis des autres sérines protéases de la coagulation et de la fibrinolyse sont tous supérieurs à 33 µM.

### EXEMPLE 9 : Composition pharmaceutique : Soluté injectable

### Soluté injectable :

Composé de l'exemple 1 : 5 mg
Eau distillée pour préparation injectable, QSP : 25 ml

| Comprimé : | |
|---|---|
| Formule de préparation pour 1000 comprimés | |
| Composé de l'exemple 1 | 5 g |
| Amidon de blé | 10 g |
| Amidon de maïs | 10 g |
| Lactose | 60 g |
| Stéarate de magnésium | 2 g |
| Silice, Hydroxypropylcellulose | 2 g |

Une enveloppe supplémentaire assurera la gastrorésistance de la forme galénique.

## Revendications

1. Composés de formule (I) H-D-Phe-A1-Arg-Pro-(Gly)4-Asn-Gly-Asp-Phe-Glu-Abo-Ile-Pro-Glu-Glu-A2-Leu-D-Glu-OH (I) dans laquelle :
A₁ représente un résidu proline (Pro), octahydroindole-2-carbonyle (Oic), 2-azabicyclo[2.2.1]heptane-3-carbonyle (Abh) ou 2-azabicyclo [2.2.2]octane-3-carbonyle (Abo),
A₂ représente un résidu phénylalanine substitué en para ou en méta par un groupement PO₃H₂(Phe(pPO₃H₂), Phe(mPO₃H₂)),
leurs sels d'addition à un acide ou à une base pharmaceutiquement acceptable, chaque amino-acide de la séquence peptidique étant optiquement pur et le carbone _ de chaque amino-acide ayant la configuration D ou L.

2. Composés de formule (I) selon la revendication 1 tels que A₁ représente un résidu proline (Pro).

3. Composés de formule (I) selon la revendication 1 tels que A₂ représente un résidu (pPO₃H₂)phénylalanine.

4. Composé de formule (I) selon la revendication 1 qui est le

H-D-Phe-Pro-Arg-Pro-(Gly)4-Asn-Gly-Asp-Phe-Glu-Abo-Ile-Pro-(Glu)2-Phe(pPO3H2)-Leu-D-Glu-OH.

5. Composé de formule (I) selon la revendication 1 qui est le

H-D-Phe-Oic-Arg-Pro-(Gly)4-Asn-Gly-Asp-Phe-Glu-Abo-Ile-Pro-(Glu)2-Phe(pPO3H2)-Leu-D-Glu-OH.

6. Procédé de préparation des commposés de formule (I) selon la revendication 1 caractérisé en ce qu'ils peuvent être obtenus par synthèse séquentielle sur phase solide à partir d'amino-acides protégés, par synthèse à partir de fragments peptidiques en solution, éventuellement par combinaison de ces deux techniques,
dérivés de formule (I) que l'on transforme, si nécessaire, en leurs sels d'addition à un acide ou à une base pharmaceutiquement acceptable.

7. Compositions pharmaceutiques contenant comme principe actif au moins un composé selon l'une quelconque des revendications 1 à 5, seul ou en combinaison avec un ou plusieurs excipients ou véhicules inertes, non toxiques, pharmaceutiquement acceptables.

8. Compositions pharmaceutiques selon la revendication 7 contenant au moins un principe actif selon l'une quelconque des revendications 1 à 5 utiles comme anticoagulants, dans le traitement des thromboses veineuses aigües, de l'embolie pulmonaire, dans l'embolie artérielle des extrêmités, de l'infarctus du myocarde, de l'athérosclérose et des manifestations thromboemboliques, ainsi que pour maintenir l'homéostasie sanguine en particulier dans la circulation extracorporelle.

## Patentansprüche

1. Verbindungen der Formel (1) H-D-Phe-A1-Arg-Pro-(Gly)4-Asn-Gly-Asp-Phe-Glu-Abo-Ile-Pro-Glu-Glu-A2-Leu-D-Glu-OH (I) in der:
A₁ einen Prolinrest (Pro), Octahydroindol-2-carbonylrest (Oic), 2-Azabicyclo[2.2.1]heptan-3-carbonylrest (Abh) oder 2-Azabicyclo[2.2.2]octan-3-carbonylrest (Abo) und
A₂ einen Phenylalaninrest, der in der para- oder meta-Stellung durch eine Gruppe PO₃H₂(Phe(pPO₃H₂), Phe(mPO₃H₂)) substituiert ist,
bedeuten, deren Additionssalze mit einer pharmazeutisch annehmbaren Säure oder Base, wobei jede Aminosäure der Peptidsequenz optisch rein ist und das α-Kohlenstoffatom einer jeden Aminosäure die Konfiguration D oder L besitzt.

2. Verbindungen der Formel (I) nach Anspruch 1, worin A₁ einen Prolinrest (Pro) bedeutet.

3. Verbindungen der Formel (I) nach Anspruch 1, worin A₂ einen (pPO₃H₂)-Phenylalaninrest bedeutet.

4. Verbindung der Formel (I) nach Anspruch 1, nämlich H-D-Phe-Pro-Arg-Pro-(Gly)₄-Asn-Gly-Asp-Phe-Glu-Abo-Ile-Pro-(Glu)₂-Phe(pPO₃H₂)-Leu-D-Glu-OH.

5. Verbindung der Formel (I) nach Anspruch 1, nämlich H-D-Phe-Oic-Arg-Pro-(Gly)₄-Asn-Gly-Asp-Phe-Glu-Abo-Ile-Pro-(Glu)₂-Phe(pPO₃H₂)-Leu-D-Glu-OH.

6. Verfahren zur Herstellung der Verbindungen der Formel (I) nach Anspruch 1, dadurch gekennzeichnet, daß sie durch eine sequentielle Synthese auf fester Phase ausgehend von geschützten Aminosäuren, durch Synthese in Lösung ausgehend von Peptidfragmenten oder gegebenenfalls durch eine Kombination dieser beiden Methoden hergestellt werden können,
wonach man die Derivate der Formel (I) erforderlichenfalls in ihre Additionssalze mit einer pharmazeutisch annehmbaren Säure oder Base umwandelt.

7. Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien oder Bindemitteln.

8. Pharmazeutische Zubereitungen nach Anspruch 7 enthaltend mindestens einen Wirkstoff nach einem der Ansprüche 1 bis 5 zur Verwendung als Antikoagulantien, bei der Behandlung von akuten Venenthrombosen, von Lungenembolie, von Arterienembolie der Extremitäten, von Myokardinfarkt, Atherosklerose und thromboembolischen Manifestationen sowie zur Aufrechterhaltung der Bluthämostasie insbesondere bei der künstlichen Durchblutung.

## Claims

1. Compounds of formula (I) 
H-D-Phe-A1-Arg-Pro-(Gly)4-Asn-Gly-Asp-Phe-Glu-Abo-Ile-Pro-Glu-
Glu-A2-Leu-D-Glu-OH (I)
 wherein:
A₁ represents a proline (Pro), octahydroindole-2-carbonyl (Oic), 2-azabicyclo[2.2.1]heptane-3-carbonyl (Abh) or 2-azabicyclo[2.2.2]octane-3-carbonyl (Abo) residue,
A₂ represents a phenylalanine residue substituted in the para or meta position by a PO₃H₂ group (Phe(pPO₃H₂), Phe(mPO₃H₂)),
their addition salts with a pharmaceutically acceptable acid or base, each amino acid of the peptide sequence being optically pure and the a carbon of each amino acid being in the D or L configuration.

2. Compounds of formula (I) according to claim 1 wherein A₁ represents a proline (Pro) residue.

3. Compounds of formula (I) according to claim 1 wherein A₂ represents a (pPO₃H₂)phenylalanine residue.

4. Compound of formula (I) according to claim 1 which is H-D-Phe-Pro-Arg-Pro-(Gly)₄-Asn-Gly-Asp-Phe-Glu-Abo-Ile-Pro-(Glu)₂-Phe(pPO₃H₂)-Leu-D-Glu-OH.

5. Compound of formula (I) according to claim 1 which is H-D-Phe-Oic-Arg-Pro-(Gly)₄-Asn-Gly-Asp-Phe-Glu-Abo-Ile-Pro-(Glu)₂-Phe(pPO₃H₂)-Leu-D-Glu-OH.

6. Process for the preparation of the compounds of formula (I) according to claim 1 characterised in that they may be obtained by solid-phase sequential synthesis starting from protected amino acids, or by synthesis starting from peptide fragments in solution, or optionally by a combination of those two techniques,
which compounds of formula (I) are converted, if necessary, into their addition salts with a pharmaceutically acceptable acid or base.

7. Pharmaceutical compositions comprising as active ingredient at least one compound according to any one of claims 1 to 5, on its own or in combination with one or more inert, non-toxic pharmaceutically acceptable excipients or carriers.

8. Pharmaceutical compositions according to claim 7 comprising at least one active ingredient according to any one of claims 1 to 5 for use as anticoagulants, in the treatment of acute venous thromboses, pulmonary embolism, arterial embolism of the extremities, myocardial infarction, atherosclerosis and thromboembolic events, and in maintaining blood homeostasis especially in extracorporeal circulation.
